# FASCICULE DE BREVET EUROPEEN

(11) **EP 2 766 471 B1**
(45) Date de publication et mention de la délivrance du brevet: **23.08.2017**
(21) Numéro de dépôt: 12775738.3
(22) Date de dépôt: 26.09.2012
(51) Int. Cl.: C12N 1/14, C12N 9/42, C12N 1/38, C12N 9/24

(54) **PROCEDE DE PRODUCTION DE CELLULASES EN CONTINU PAR UN CHAMPIGNON FILAMENTEUX UTILISANT UN SUBSTRAT CARBONE ISSU D'UN PRETRAITEMENT ACIDE**
VERFAHREN ZUR KONTINUIERLICHEN HERSTELLUNG VON CELLULASEN DURCH FADENPILZE MIT EINEM KOHLENSTOFFSUBSTRAT AUS EINER SÄUREVORBEHANDLUNG
METHOD FOR THE CONTINUOUS PRODUCTION OF CELLULASES BY A FILAMENTOUS FUNGUS USING A CARBON SUBSTRATE OBTAINED FROM AN ACID PRETREATMENT

(30) Priorité: 14.10.2011 FR 1103149
(43) Date de publication de la demande: 20.08.2014
(73) Titulaire: IFP Énergies nouvelles, 92852 Rueil-Malmaison Cedex (FR)
(72) Inventeur: BEN CHAABANE, Fadhel, F-75020 Paris (FR); CHAUSSEPIED, Bernard, F-28130 Hanches (FR)
(86) Numéro de dépôt international: PCT/FR2012/000381
(87) Numéro de publication internationale: WO 2013/054005

(56) Documents cités:
- WO-A1-2009/026716
- WO-A1-2012/007650
- T. M. PAKULA: "The effect of specific growth rate on protein synthesis and secretion in the filamentous fungus Trichoderma reesei", MICROBIOLOGY, vol. 151, no. 1, 1 janvier 2005 (2005-01-01), pages 135-143, XP055032610, ISSN: 1350-0872, DOI: 10.1099/mic.0.27458-0
- SCHAFNER D W ET AL: "CELLULASE PRODUCTION IN CONTINUOUS CULTURE BY TRICHODERMA-REESEI ON XYLOSE-BASED MEDIA", BIOTECHNOLOGY AND BIOENGINEERING, WILEY & SONS, HOBOKEN, NJ, US, vol. 39, no. 8, 1 janvier 1992 (1992-01-01), pages 865-869, XP002628529, ISSN: 0006-3592, DOI: 10.1002/BIT.260390808
- HENDY N A ET AL: "Enhanced cellulase production in fed-batch culture of Trichoderma reesei C30", ENZYME AND MICROBIAL TECHNOLOGY, STONEHAM, MA, US, vol. 6, no. 2, 1 février 1984 (1984-02-01) , pages 73-77, XP023678270, ISSN: 0141-0229, DOI: 10.1016/0141-0229(84)90038-3 [extrait le 1984-02-01]
- XIONG H ET AL: "Xylanase production by Trichoderma reesei Rut C-30 grown on L-arabinose-rich plant hydrolysates", BIORESOURCE TECHNOLOGY, ELSEVIER BV, GB, vol. 96, no. 7, 1 mai 2005 (2005-05-01), pages 753-759, XP025313240, ISSN: 0960-8524, DOI: 10.1016/J.BIORTECH.2004.08.007 [extrait le 2005-05-01]
- RYU D ET AL: "STUDIES ON QUANTITATIVE PHYSIOLOGY OF TRICHODERMA-REESEI WITH 2 STAGE CONTINUOUS CULTURE FOR CELLULASE PRODUCTION", BIOTECHNOLOGY AND BIOENGINEERING, vol. 21, no. 11, 1979, pages 1887-1904, XP055032623, ISSN: 0006-3592
- LO C M ET AL: "Cellulase production by continuous culture of Trichoderma reesei Rut C30 using acid hydrolysate prepared to retain more oligosaccharides for induction", BIORESOURCE TECHNOLOGY, ELSEVIER BV, GB, vol. 101, no. 2, 1 janvier 2010 (2010-01-01), pages 717-723, XP026662968, ISSN: 0960-8524, DOI: 10.1016/J.BIORTECH.2009.08.056 [extrait le 2009-09-22]
- CHAUDHURI B K ET AL: "Comparison of growth and maintenance parameters for cellulase biosynthesis by Trichoderma reesei-C5 with some published data", ENZYME AND MICROBIAL TECHNOLOGY, STONEHAM, MA, US, vol. 16, no. 12, 1 décembre 1994 (1994-12-01), pages 1079-1083, XP023679737, ISSN: 0141-0229, DOI: 10.1016/0141-0229(94)90146-5 [extrait le 1994-12-01]
- JU ET AL: "Wastepaper hydrolysate as soluble inducing substrate for cellulase production in continuous culture of Trichoderma reesei", BIOTECHNOLOGY PROGRESS, AMERICAN INSTITUTE OF CHEMICAL ENGINEERS, US, vol. 15, 1 janvier 1999 (1999-01-01), pages 91-97, XP008148771, ISSN: 8756-7938, DOI: 10.1021/BP980116N
- INGRID PERSSON ET AL: "Fungal cellulolytic enzyme production: A review", PROCESS BIOCHEMISTRY, vol. 26, no. 2, 1 avril 1991 (1991-04-01), pages 65-74, XP055032619, ISSN: 1359-5113, DOI: 10.1016/0032-9592(91)80019-L

## Description

### DOMAINE DE L'INVENTION

La présente invention concerne la production de cellulases et d'hémicellulases, notamment dans le cadre de la production d'éthanol à partir de matériaux ligno-cellulosiques. En particulier, la présente invention concerne un procédé de production de cellulase en continu à partir d'un champignon filamenteux.

### ART ANTÉRIEUR

La mise au point de procédés économiquement viables de production de biocarburants de 2ème génération est aujourd'hui un vaste sujet d'actualité. Ces derniers sont produits à partir de biomasse lignocellulosique et posent moins de problèmes de concurrence d'usage des terres agricoles avec l'alimentaire, par rapport aux biocarburants dits de première génération qui sont produits à partir de canne à sucre, maïs, blé ou betterave.

La biomasse ligno-cellulosique se caractérise par une structure complexe constituée de trois principales fractions: la cellulose, les hémicelluloses et les lignines. De façon classique, le procédé de transformation en éthanol comprend plusieurs étapes. Le prétraitement permet de rendre la cellulose accessible aux enzymes qui sont des cellulases. L'étape d'hydrolyse enzymatique permet la transformation de la cellulose en glucose qui est ensuite transformée en éthanol lors de l'étape de fermentation par, en général, la levure *Saccharomyces cerevisiae.* Enfin, l'étape de distillation va permettre de séparer et récupérer l'éthanol du moût de fermentation.

Les différentes études technico-économiques démontrent que la réduction du coût des cellulases est un des points-clés des procédés de production biologique d'éthanol à partir des matières premières lignocellulosiques. A l'heure actuelle, les cellulases industrielles sont principalement produites par un champignon filamenteux, *Trichoderma reesei,* en raison de son fort pouvoir de sécrétion. *Trichoderma reesei* est le microorganisme le plus utilisé pour la production de cellulases. Les souches sauvages ont la faculté d'excréter, en présence d'un substrat inducteur, la cellulose par exemple, le cocktail enzymatique considéré comme le mieux adapté à l'hydrolyse de la cellulose. Les enzymes du cocktail enzymatique contiennent trois grands types d'activités : les endoglucanases, les exoglucanases et les cellobiases. D'autres protéines possédant des propriétés indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques.
La régulation des gènes de cellulases sur différentes sources de carbone a été étudiée dans le détail. Elles sont induites en présence de cellulose, de ses produits d'hydrolyse (exemple: cellobiose) ou de certains oligosaccharides comme le lactose ou le sophorose (limén et al., 1997; Appl.Environ. Microbiol. 63: 1298-1306).
Les techniques de génétique classique par mutation ont permis la sélection de souches de *Trichoderma reesei* hyperproductrices de cellulases telles que les souches MCG77 (Gallo - brevet US 4275 167), MCG 80 (Allen, A.L. et Andreotti, R.E., Biotechnol- Bioengi 1982, 12, 451-459 1982), RUT C30 (Montenecourt, B.S. et Eveleigh, D.E., Appl. Environ. Microbiol. 1977, 34, 777-782) et CL847 (Durand et al, 1984, Proc. Colloque SFM "Génétique des microorganismes industriels". Paris. H. HESLOT Ed, pp 39-50).
Le procédé de production de cellulases par *Trichoderma reesei* a fait l'objet d'améliorations importantes en vue de l'extrapolation à l'échelle industrielle. La stratégie qui est appliquée industriellement est de faire une croissance rapide du champignon jusqu'à une concentration donnée dans une étape appelée phase de croissance dudit champignon, puis d'induire la production de cellulases à partir dudit champignon afin de maximiser la productivité et le rendement dans une étape appelée phase et de production. Ladite phase de croissance est en général réalisé dans un réacteur fermé, c'est-à-dire en mode "batch" selon la terminologie anglo-saxonne. Ladite phase de production, est en général réalisée dans un réacteur à alimentation continue au cours de laquelle aucun soutirage du contenu du réacteur n'est effectué, c'est à dire en mode "fed batch" selon la terminologie anglo-saxonne. Pour obtenir de bonnes productivités en enzymes, il est nécessaire d'apporter une source de carbone rapidement assimilable pour la croissance de *Trichoderma reesei* dans la phase de croissance et un substrat inducteur qui permette l'expression des cellulases et la sécrétion dans le milieu de culture dans la phase de production. La cellulose peut jouer ces deux rôles ; cependant, elle est difficile d'utilisation au stade industriel et a été remplacée par des sources de carbone solubles tel que le lactose, qui permet l'expression des cellulases. D'autres sucres solubles comme le cellobiose et le sophorose ont été décrits comme substrats inducteurs, mais ils sont trop onéreux pour être utilisés au stade industriel. Cependant, les productions de cellulases par *Trichoderma reesei,* avec des substrats solubles, sont très inférieures à celles obtenues sur cellulose en mode "batch". Ceci est dû à l'effet répresseur des sucres facilement assimilables, à forte concentration.
L'alimentation en continu en mode "fed-batch" des substrats carbonés inducteurs solubles a permis de lever la répression catabolique en limitant la concentration résiduelle de substrat carboné dans les cultures et en optimisant la quantité de sucre permettant d'obtenir un meilleur rendement et une meilleure productivité enzymatique. Par exemple, le brevet FR-B-2 881 753 décrit un procédé de production de cellulases comprenant deux étapes :
- Une phase de croissance en mode "batch" où il est nécessaire d'apporter une source de carbone rapidement assimilable pour la croissance de Trichoderma reesei puis
- Une phase de production en mode "fed-batch" utilisant un substrat inducteur tel que par exemple le lactose qui permet l'expression des cellulases et la sécrétion dans le milieu de culture. L'alimentation en substrat carboné soluble se fait en continu, à un débit spécifique optimal appliqué, exprimé en mg de substrat par gramme en poids sec de champignon filamenteux et par heure, compris entre 35 et 45 mg.g⁻¹.h⁻¹.
Dans ce brevet, l'étape de production des protéines n'est pas mis en oeuvre au delà d'environ 170 h. Ce protocole permet d'aboutir à une concentration en protéines de l'ordre de 35 à 40 g/L avec une productivité de l'ordre de 0,2 g.L⁻¹.h⁻¹.
Cependant, le réacteur doit être nettoyé et une nouvelle chaîne d'ensemencement doit être réalisée. L'inconvénient de ce mode opératoire est une productivité trop faible qui fait augmenter l'investissement initial en nombre de fermenteurs de production d'enzymes. La concentration en protéines obtenue est également peu élevée et nécessite souvent une étape de concentration après filtration du mycellium. Tout ceci contribue à rendre le procédé de production d'éthanol de deuxième génération peu compétitif. WO2009/026716 décrit une culture en continu de Trichoderma pour la production de cellulase et d'hémicellulase. La source de carbone est un mélange de sucres dérivés d'hémicellulose et de sucres inducteurs de cellulase. Le taux de dilution de la culture en continu est de 0.025 h⁻¹.

Un objet de la présente invention est de fournir un procédé de production de cellulases et d'hémicellulases utilisant au moins un substrat carboné inducteur spécifique issu du prétraitement acide d'un substrat lignocellulosique permettant d'augmenter voire de doubler la productivité et la concentration en cellulases et hémicellulases produites par rapport aux procédés de l'art antérieur, et de produire ces cellulases en continu pendant une durée accrue. Le procédé selon la présente invention permet d'augmenter voire de doubler la productivité et la concentration en cellulases et hémicellulases produites tout en maintenant le rendement de production de cellulases par rapport au substrat carboné utilisé constant par rapport aux procédés de l'art antérieur.

### RÉSUMÉ ET INTÉRÊT DE L'INVENTION

La présente invention concerne un procédé de production de cellulases et hémicellulases par une souche appartenant à un champignon filamenteux, en bioréacteur agité et aéré comprenant au moins deux phases :
- une phase a) de croissance de ladite souche en présence d'au moins un substrat carboné de croissance en réacteur fermé, ladite phase de croissance étant réalisée avec une concentration en substrat carboné de croissance comprise entre 10 et 90 g/L
- une phase b) continue de production de cellulases dans laquelle au moins un substrat carboné inducteur est alimenté à un débit d'alimentation au moins constant pendant une durée au moins supérieure à 200h, ledit substrat carboné inducteur étant au moins une solution aqueuse d'hydrolysat hémicellulosique issu d'un prétraitement acide d'un substrat lignocellulosique, ladite solution aqueuse d'hydrolysat hémicellulosique ne subissant pas de stérilisation préalable et pas de rectification de pH, ledit pH de la solution aqueuse étant compris entre 0,5 et 3, la masse du volume réactionnel étant maintenu constant par soutirage d'une fraction dudit volume réactionnel, ladite phase b) opérant à un taux de dilution compris entre 0,001 et 0,008 h⁻¹,
dans lequel le substrat carboné inducteur utilisé dans la phase b) est une solution aqueuse d'hydrolysat hémicellulosique issu d'un prétraitement acide d'un substrat lignocellulosique en mélange avec au moins un autre substrat carboné choisi parmi des sucres inducteurs ou non inducteurs,
dans lequel le débit d'alimentation dudit substrat carboné inducteur est compris entre 35 et 140 mg de substrat carboné inducteur par gramme en poids sec de souche et par heure.

### Avantage de l'invention

Un avantage de la présente invention est de permettre d'améliorer la productivité et la concentration en protéines produites sur une durée de fonctionnement accrue. En particulier, le procédé selon l'invention permet l'obtention d'une concentration en protéines supérieure à 100g.L-1. Ces performances ont été maintenues expérimentalement en mode continu pendant plus de 400 h.
La forte productivité obtenue permet de réduire les coûts d'investissements en bioréacteur. La durée prolongée permet de réduire le temps consacré au nettoyage des bioréacteurs et aux chaines d'ensemencement. La forte concentration en cellulases permet de réduire les coûts de post-traitement.

Un autre avantage du procédé continu selon l'invention est qu'il nécessite un faible k_{L}a du fait, à la fois de l'application d'un faible taux de dilution dans la phase b) continue de production et de l'utilisation d'une solution de substrat carboné inducteur spécifique dans ladite phase b), ce qui permet de maintenir une faible viscosité dans le milieu réactionnel.

### DESCRIPTION DÉTAILLÉE DE L'INVENTION

Le procédé selon l'invention opère de préférence à un pH compris entre 3 et 6, à une température comprise entre 20 et 35 °C, à une vvm, c'est-à-dire à un taux d'aération exprimé en volume d'air, dans les conditions normales de température et de pression, par volume de milieu réactionnel et par minute comprise entre 0,3 et 1,5 min-1, de préférence entre 0,3 et 1 min-1 et avec une agitation permettant d'obtenir une pression partielle en oxygène dans le milieu réactionnel comprise entre 20% et 60% et de préférence comprise entre 20 et 40%.
De préférence, le procédé selon l'invention opère à une vvm de 0.5 min-1 et avec une agitation permettant de réguler la pression partielle en oxygène à 30 %.

Conformément à l'invention, ledit procédé comprend une phase a) de croissance de la souche appartenant à un champignon filamenteux, de préférence le champignon *Trichoderma reesei,* en présence d'au moins un substrat carboné de croissance en réacteur fermé, ladite phase de croissance étant réalisée avec une concentration en substrat carboné de croissance comprise entre 10 et 90 g/L.

Ladite souche mise en oeuvre dans le procédé selon l'invention est une souche d'un champignon filamenteux appartenant de préférence aux genres *Trichoderma, Aspergillus, Penicillium ou Schizophyllum,* et de manière préférée ladite souche appartient à l'espèce *Trichoderma reesei.*

La souche utilisée appartenant de préférence à l'espèce *Trichoderma reesei,* peut avantageusement être modifiée pour améliorer les enzymes cellulolytiques et/ou hémicellulolytiques par des procédés de mutation-sélection, comme par exemple la souche IFP CL847. Une souche améliorée par les techniques de recombinaison génétique peut également être utilisée. Ladite souche est cultivée en réacteurs agités et aérés dans des conditions compatibles avec sa croissance et la production de cellulases. D'autres souches de microorganismes produisant des cellulases selon des processus similaires à ceux utilisés pour *Trichoderma* peuvent être utilisées.
De manière très préférée, la souche utilisée est une souche de *Trichoderma reesei* modifiée par mutation, sélection ou recombinaison génétique.
La souche peut avantageusement être choisie parmi les souches CL847, RutC30, MCG77, ou MCG80.
Le substrat carboné de croissance utilisé dans ladite phase de croissance est avantageusement choisi parmi les sucres solubles industriels, et de préférence parmi le glucose, le lactose, le xylose, les résidus obtenus après fermentation éthanolique des sucres monomères des hydrolysats enzymatiques de substrat lignocellulosique et les extraits de la fraction hémicellulosique sous forme de monomères provenant de substrat lignocellulosique prétraité, utilisé seul ou en mélange.
Selon sa nature, ledit substrat carboné est avantageusement introduit dans le réacteur avant stérilisation dudit réacteur ou est stérilisé séparément et introduit dans le réacteur préalablement stérilisé.
De préférence, la concentration en substrat carboné de croissance est comprise entre 30 et 70 g/L.
De préférence, la phase a) de croissance est réalisée sur une durée comprise entre 30 et 70h et de préférence entre 40 et 60h.
De préférence, la phase a) de croissance opère à un pH de 4,8 et à une température de 27°C.
Conformément à l'invention, ledit procédé comprend une phase b) continue de production de cellulases dans laquelle au moins un substrat carboné inducteur est alimenté à un débit d'alimentation au moins constant, pendant une durée au moins supérieure à 200h, ledit substrat carboné étant au moins une solution aqueuse d'hydrolysat hémicellulosique issu d'un prétraitement acide d'un substrat lignocellulosique, ladite solution aqueuse d'hydrolysat hémicellulosique ne subissant pas de stérilisation préalable et pas de rectification de pH, ledit pH de la solution aqueuse étant compris entre 0,5 et 3, la masse du volume réactionnel étant maintenu constant par soutirage d'une fraction dudit volume réactionnel, ladite phase b) opérant à un taux de dilution compris entre 0,001 et 0,008 h⁻¹.

Ladite phase continue est avantageusement réalisée dans un réacteur à alimentation continue au cours de laquelle une fraction du volume réactionnel est soutirée de manière à maintenir la masse du volume réactionnel constante. Ladite phase continue est appelé mode "chemostat" selon la terminologie anglo-saxonne.

Le substrat lignocellulosique permettant d'obtenir la solution aqueuse d'hydrolysat hémicellulosique utilisée dans la phase b) du procédé selon l'invention est une source d'hydrates de carbones composée de trois principaux constituants : la cellulose (35 à 50%), les hémicelluloses (20 à 30%) qui sont des polysaccharides essentiellement constitués de pentoses et d'hexoses et la lignine (15 à 25%) qui est une macromolécule de structure complexe et de haut poids moléculaire, composé d'alcools aromatiques reliés par des liaisons éther. Ledit substrat est avantageusement choisi parmi les pailles, le bois, les cultures forestières, les résidus de plantes alcooligènes, sucrières et céréalières, les résidus de l'industrie papetière et les produits de transformation des matériaux lignocellulosique.

Le prétraitement acide subi par ledit substrat lignocellulosique est mis en oeuvre selon les prétraitements acides connus de l'homme du métier. De préférence, le prétraitement acide est une hydrolyse acide, une cuisson acide ou une explosion à la vapeur avec imprégnation préalable dudit substrat lignocellulosique avec une solution aqueuse d'acide sulfurique.

La solution aqueuse d'hydrolysat hémicellulosique ainsi obtenue présente un pH compris entre 0,5 et 3 et est utilisée sans étape de stérilisation ni de rectification du pH.

De préférence, ladite solution aqueuse d'hydrolysat hémicellulosique présente un pH compris entre 0,5 et 2.
Le substrat carboné inducteur utilisé dans la phase b) du procédé selon l'invention est avantageusement une solution aqueuse d'hydrolysat hémicellulosique issu d'un prétraitement acide d'un substrat lignocellulosique seule ou en mélange avec au moins un autre substrat carboné n'ayant pas subi de stérilisation.
De préférence, lesdits substrats carbonés sont choisis parmi des sucres inducteurs ou non inducteurs, de manière préférée choisis parmi le lactose, le glucose, le cellobiose et le xylose, pris seuls ou en mélange.

Lesdits substrats sont dissouts dans ladite solution aqueuse d'hydrolysat hémicellulosique.
Dans le cas où le substrat carboné inducteur est une solution aqueuse d'hydrolysat hémicellulosique issu d'un prétraitement acide d'un substrat lignocellulosique en mélange avec au moins un autre substrat carboné n'ayant pas subi de stérilisation, ledit substrat carboné inducteur présente une concentration comprise entre 200 et 600 g/L selon le degré de solubilité des substrats carbonés utilisés.
Dans le cas où le substrat carboné inducteur est une solution aqueuse d'hydrolysat hémicellulosique issu d'un prétraitement acide d'un substrat lignocellulosique seule, ledit substrat carboné inducteur présente une concentration comprise entre 40 et 400 g/L après avoir éventuellement été concentrée.

L'utilisation dudit substrat carboné inducteur spécifique issu du prétraitement acide d'un substrat lignocellulosique permet la mise en oeuvre de la phase b) de production de cellulases sur une durée accrue, de préférence supérieure à 200h, par rapport aux procédé de l'art antérieur.

Conformément à l'invention, ledit substrat carboné inducteur est alimenté à un débit d'alimentation au moins constant. De préférence, le débit d'alimentation dudit substrat carboné inducteur est compris entre 35 et 140 et de préférence entre 35 et 60 mg de substrat carboné inducteur par gramme en poids sec de souche et par heure.

De manière préférée, le débit d'alimentation est graduellement augmenté dans la phase b), de manière plus préférée graduellement augmenté jusqu'à être doublé dans les premières heures de mise en oeuvre de la phase b), de préférence après au moins 24h et de manière préférée après au moins 48h de mise en oeuvre de la phase b).

Conformément à l'invention, la durée de la phase b) continue de production de cellulases est au moins supérieure à 200h, de préférence au moins supérieure à 300h et de manière préféré au moins supérieure à 400h.

Dans la phase b), la masse du volume réactionnel est maintenue constante par soutirage d'une fraction dudit volume réactionnel.

De préférence, le soutirage est réalisé selon les méthodes de soutirage connues de l'homme du métier telles que par exemple grâce à un système de régulation et à une pompe de soutirage commandable.
De préférence, le débit de soutirage est au moins égal au débit d'alimentation dans la phase b).
Conformément à l'invention, le taux de dilution, défini comme le ratio du débit de soutirage sur le volume réactionnel du réacteur lors de la phase b) continue de production, est avantageusement compris entre 0,001h-1 et 0,008 h-1 et de manière préférée entre 0,002 et 0,008h-1. Le taux de dilution très préféré est de 0,004 h-1. De préférence, la phase b) opère à un pH compris entre 3 et 5,5 et à une température comprise entre 20 et 30°C.
Une phase optionnelle a') de production réalisée dans un réacteur à alimentation continue d'au moins un substrat carboné inducteur, au cours de laquelle aucun soutirage du contenu du fermenteur n'est effectué, c'est à dire en mode "fed batch", est avantageusement mise en oeuvre entre la phase a) et la phase b).
La mise en oeuvre de ladite phase a') permet de ne pas soutirer de fraction du volume réactionnel contenant la souche de champignon filamenteux alors que les concentrations en protéines produites sont encore faibles.
Ledit substrat carboné inducteur utilisé dans la phase a') est identique au substrat carboné inducteur utilisé dans la phase b) de production.
De préférence, le débit d'alimentation dudit substrat carboné inducteur est compris entre 35 et 140 et de préférence entre 35 et 60 mg de substrat carboné inducteur par gramme en poids sec de souche et par heure. Ledit débit d'alimentation est maintenu constant pendant toute la durée de la phase a').
De préférence, la phase a') est mise en oeuvre pendant une durée comprise entre 50 et 150 h et de préférence entre 70 et 130h.
De préférence, la phase a') opère à un pH compris entre 3 et 5,5 et à une température comprise entre 20 et 30°C.
Le procédé selon la présente invention permet d'augmenter voire de doubler la productivité ainsi que la concentration en cellulases et hémicellulases produites par rapport aux procédés de l'art antérieur, et de produire ces cellulases en continu pendant une durée accrue.

### EXEMPLES

### Exemple 1 : non conforme

L'exemple 1 présente une culture utilisant les conditions de référence du brevet FR-B-2 881 753. L'exemple 1 illustre un procédé de production de cellulases et hémicellulases comportant une phase de croissance et une phase de production en mode "fed batch" mise en oeuvre pendant 167 h
La production de cellulases et hémicellulases est effectuée en réacteur agité mécaniquement de 3 L. Le milieu minéral a la composition suivante : KOH 1,66 g./L, H₃PO₄ 85 % 2 ml/L, (NH₄)₂SO₄ 2,8 g/L, MgSO₄, 7 H₂O 0,6 g/L, CaCL₂ 0,6 g/L, MnSO₄ 3,2 mg/L, ZnSO₄, 7 H₂O 2,8 mg/L, CoCl₂ 10 4,0 mg/L, FeSO₄, 7 H₂O 10 mg/L, Corn Steep 1,2 g/L, anti-mousse 0,5 mL/L.
Une préculture liquide de la souche de *Trichoderma reesei* CL847 est réalisée. Le milieu minéral de la préculture, est identique à celui du réacteur mis à part l'addition de phtalate de potassium à 5 g/L pour tamponner le pH. La croissance du champignon en préculture est faite en utilisant le glucose comme substrat carboné, à la concentration de 30 g.L-1. La croissance de l'inoculum dure de 2 à 3 jours et est effectuée à 28 °C dans un incubateur agité à pression atmosphérique.
Le réacteur contenant le milieu minéral est stérilisé à 120 °C pendant 20 minutes, la source carbonée glucose est stérilisée à part à 120°C pendant 20 minutes puis ajoutée stérilement dans le réacteur de façon à avoir une concentration finale de 30 g/L. Le réacteur est ensemencé à 10% (v/v) avec ladite préculture liquide de la souche de *Trichoderma reesei* CL847 dès que la concentration résiduelle en glucose dans la préculture est inférieure à 15 g/L.

L'expérience effectuée en bioréacteur comporte deux phases :
- Une phase de croissance sur substrat carboné glucose (concentration initiale = 30 g/L) à une température de 27 °C et un pH de 4,8 (régulé par de l'ammoniaque 5,5 M). L'aération est de 0,5 vvm et l'agitation est augmentée entre 200 et 800 rpm en fonction de la pO₂ (pression en oxygène dissous), qui est régulée à 30 %.
- Une phase de production de protéines en mode "fed batch". Après 30 heure, une solution de substrat carboné lactose à 250 g.L-1 est injectée en continu au débit de 4 mL/h soit à 35 mg de lactose par g de souche de *Trichoderma reesei* CL847 et par heure jusqu'à 167 heures. La température est baissée à 25 °C et le pH à 4 jusqu'à la fin de la culture. Le pH est régulé par addition d'une solution d'ammoniaque à 5,5 N qui apporte l'azote nécessaire à la synthèse des cellulases et hémicellulases excrétées. La teneur en oxygène dissous est maintenue à 30 % par action de l'agitation

La production de cellulases est suivie par le dosage des protéines extracellulaires par la méthode de Lowry et standard BSA, après séparation du mycélium par filtration ou centrifugation. Les activités cellulolytiques déterminées sont:
- L'activité papier filtre (UPF : unité papier filtre) qui permet de doser l'activité globale du pool enzymatique endoglucanases et exoglucanases
- L'activité β-glucosidase pour les activités spécifiques.

L'activité UPF est mesurée sur papier Whatman n°1 selon la procédure recommandée par la commission biotechnologique IUPAC, à la concentration initiale de 50 g.L⁻¹ ; on détermine la prise d'essai de la solution enzymatique à analyser qui libère l'équivalent de 2 g.l⁻¹ de glucose (dosage colorimétrique) en 60 minutes. Le principe de l'activité papier filtre est de déterminer par dosage à l'acide dinitrosalicylique (DNS) la quantité de sucres réduits issue d'un papier Whatman N°1. Le substrat utilisé pour déterminer l'activité β-glucosidase est le p-nitrophenyl-ß-D-glucopyranoside (PNPG). Il est clivé par la ß-glucosidase qui libère le p-nitrophenol. Une unité d'activité ß-glucosidase est définie comme la quantité d'enzyme nécessaire pour produire 1µmol de nitrophenol à partir de PNPG par minute et est exprimée en IU/ml.
Les activités spécifiques sont obtenues en divisant les activités exprimées en IU/mL par la concentration en cellulases. Elles sont exprimées en IU.mg⁻¹.
La productivité finale est calculée en tenant compte de toute la masse des cellulases et hémicellulases produites durant la phase de production (incluant les prélèvements) et en la divisant par la durée de la phase de production et le volume utile du réacteur.
Le terme "Biomasse" caractérise la souche de *Trichoderma reesei* CL847.
Le terme "Protéine" est défini comme étant le cocktail enzymatique obtenu comprenant les cellulases et hémicellulases produites.
Les déterminations analytiques sur le moût final de l'exemple 1 donnent les résultats suivants :
Biomasse g/l : 14,4
Protéines g/l: 35,7
Productivité = 0,21 g/L/h
UPF 22,1 IU/mL
β-Glucosidase spécifique : 0,8 IU/mg

### Exemple 2: non conforme

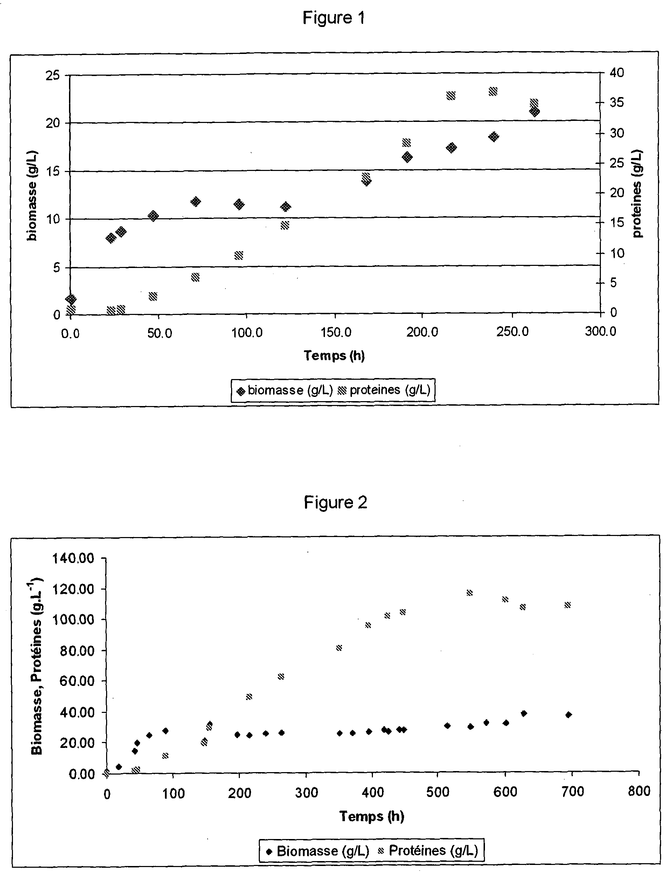

L'exemple 2 présente une culture analogue à l'exemple 1 sauf que le mode "fed-batch" est poursuivi au delà de 200h avec le même substrat d'alimentation. On constate qu'il y a un arrêt de la production de cellulases et hémicellulases après 200 h. Celles-ci commencent même à se dégrader puisque la concentration baisse de 37 g/L à 35 g/L. La biomasse augmente quant à elle durant cette période jusqu'à atteindre une concentration de 20,9 g/L. Les dosages montrent qu'il y a eu une carence en soufre.
L'évolution de la concentration en biomasse et en protéines (g/L) est représentée sur la figure 1.
Les déterminations analytiques sur le moût final donnent les résultats suivants :
Biomasse g/L : 20,9
Protéines g/L : 35,1
Productivité = 0,13 g/L/h (Elle était de 0,17 g/L/h après 216 h)
UPF 16,1 IU/mL
β-Glucosidase spécifique 0,7 IU/mg

### Exemple 3: conforme à l'invention.

L'exemple 3 est lancé dans les mêmes conditions que l'exemple 1 mais comporte 3 phases:
- Une phase a) en mode "batch" dans les mêmes conditions que l'exemple 1 mais avec une concentration en glucose de 60 g/L. Cette phase dure 50h,
- Une deuxième phase a') en mode "fed-batch". Le fedbatch est lancé au moment de l'épuisement du substrat carboné glucose avec une solution d'hydrolysat hémicellulosique issue d'une paille prétraitée par explosion à la vapeur avec imprégnation préalable de H₂SO₄ dans laquelle a été dissout du glucose et du lactose pour arriver à une concentration globale en substrat carboné de 500g/L. Cette solution n'est pas stérilisée et son pH n'est pas remonté et est de 1. Un débit de 4 mL/h (soit un flux de 35 mg de sucres par g de souche de *Trichoderma reesei* CL847 et par heure) est appliqué. Cette phase dure 100h.
- une phase b) de production continue de cellulases et hémicellulases est lancée par la suite. Le débit d'alimentation est maintenu constant à 4 mL/h durant toute l'expérience. Le réacteur est maintenu à un poids constant en soutirant continuellement le moût grâce à un système de régulation et à une pompe de soutirage commandable. Le taux de dilution est de 0,002 h-1.

Nous avons produit en continu à partir de 400h une solution enzymatique ayant une concentration supérieure à 100 g/L et avec une productivité supérieure à 0,2 g/L/h. Ceci permet donc de tripler la concentration en protéines et d'avoir une productivité supérieure à celle de l'exemple 1 (+20%).
L'évolution de la concentration en biomasse et en protéines (g/L) au cours du temps pour l'exemple 3 dans lequel la phase continue est lancée après 150 h avec un débit de 4 mL/h est représentée sur la figure 2.

Les déterminations analytiques sur le moût final donnent les résultats suivants :
Biomasse g/L : 36,7
Protéines g/L : 107,2
Productivité finale = 0,26 g/L/h
UPF 76,8 IU/mL
β-Glucosidase spécifique 1,2 IU/mg

### Exemple 4 : conforme

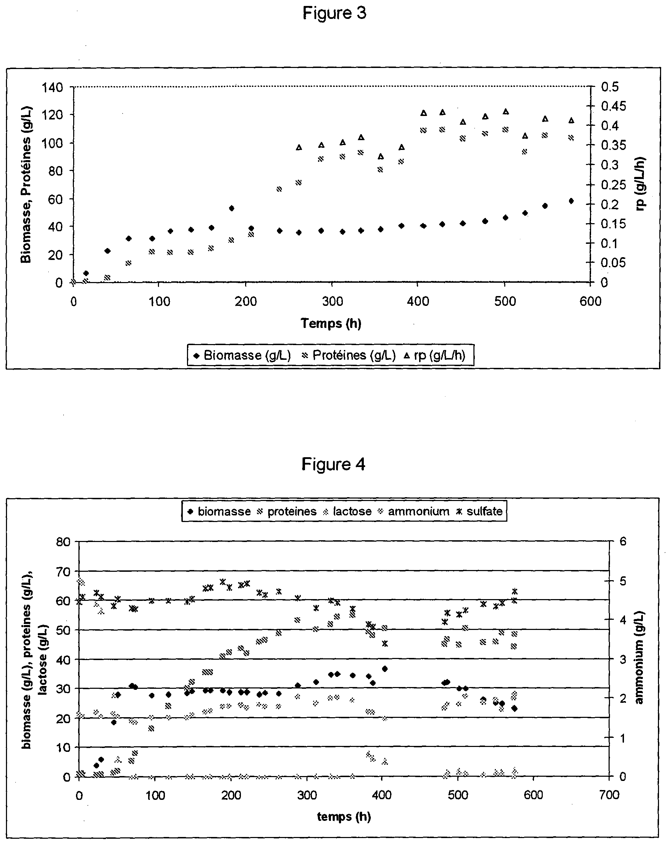

L'exemple 4 est analogue à l'exemple 3 sauf que la phase b) continue de production est lancée directement après la phase a) de croissance en mode "batch" et que le débit d'alimentation en substrat carboné inducteur dans la phase b) continue de production est augmenté graduellement de 4mL/h à 8 mL/h, c'est-à-dire augmenté de 1 mL toutes les 12 h après le lancement ladite phase b). Le substrat carboné inducteur utilisé est le même que celui de l'exemple 3, c'est-à-dire une solution d'hydrolysat hémicellulosique issue d'une paille prétraitée par explosion à la vapeur avec imprégnation préalable de H₂SO₄ dans laquelle a été dissout du glucose et du lactose. Cette solution n'est pas stérilisée et son pH n'est pas remonté et est de 1. Les conditions opératoires utilisées dans les phases a) et b) sont identiques à celles utilisées dans l'exemple 3. À l'issue de l'augmentation du débit d'alimentation en substrat carboné inducteur, le taux de dilution est de 0,004 h⁻¹. La masse du volume réactionnel est maintenue constante.
La productivité finale de l'expérience est de 0,39 g.L⁻¹.h⁻¹. Elle est presque doublée par rapport à l'exemple 1. Cela permet de réduire les coûts d'investissements. La concentration finale en protéines est presque triplée. Ce qui permet de réduire les coûts de post-traitement notamment, le cas échéant, la concentration des protéines produites. La culture ne nécessite pas un k_{L}a élevé (environ 75 h⁻¹) grâce au faible taux de dilution appliqué permettant ainsi d'avoir des faibles coûts opératoires liés à l'agitation et l'aération.
L'évolution de la productivité (rp) en cellulases et des concentrations en souches de *T.reesei* et en cellulases pour l'exemple 4 dans lequel la phase continue est lancée après 150h et le débit de fed-batch est augmenté de 4 à 8mL/h est représenté sur la figure 3.

Les déterminations analytiques sur le moût final donnent les résultats suivants
Biomasse g/L : 58,1
Protéines g/L :102,9
Productivité finale = 0,39 g/L/h
UPF 77,6 IU/mL
β-Glucosidase spécifique : 1,3 IU/mg
La productivité est presque doublée par rapport à l'exemple 1 et la concentration en protéines presque triplée. Ladite concentration est maintenue pendant plus de 300h

### Exemple 5 : non conforme

L'exemple 5 permet de montrer l'effet de la non stérilisation de la solution d'hydrolysat hémicellulosique issue d'une paille prétraitée par explosion à la vapeur avec imprégnation préalable de H₂SO₄ sur les performances du procédé.

L'exemple 5 est lancé dans les mêmes conditions que l'exemple 4 sauf que la solution d'hydrolysat est stérilisée avant utilisation. La concentration de ladite solution est de 250 g/L. La culture aboutit à une forte accumulation de la souche de *T. reesei* et à une faible production de cellulases. La demande en oxygène est très importante en fin de culture avec un k_{L}a nécessaire supérieur à 170h⁻¹.

Le rendement de production de protéine par rapport au substrat carboné est inférieur à 0,1 g/g alors qu'il est de 0,3 g/g pour les exemples 1 à 4.

Les déterminations analytiques sur le moût final donnent les résultats suivants
Biomasse : g/L : 99,8
Protéines : g/L : 24,2
UPF : 14,5 IU/mL
β-Glucosidase spécifique : 1,1 IU/mg

### Exemple 6: non-conforme

L'Exemple 6 montre l'inconvénient de la mise en oeuvre de phase continue à fort taux de dilution qui conduisent à un milieu visqueux et à des problèmes de bouchage de la pompe de soutirage à cause de la morphologie du champignon lorsqu'il est en phase de croissance. Le k_{L}a et donc le coût opératoire lié à l'agitation et l'aération du milieu est élevé.
L'exemple 6 est conduit dans les mêmes conditions que l'exemple 4 avec une première phase a) de croissance en mode "batch" qui dure 50 h et une phase b) continue de production de cellulases et hémicellulases. La solution qui aliment la phase de production est une solution d'hydrolysat hémicellulosique qui n'est pas stérilisée dans laquelle on dissout du lactose à 250 g/L. Le débit d'alimentation en substrat carboné inducteur est de 13 ml/h ce qui correspond à 54 mg de sucres par g de souche de *Trichoderma reesei* CL847 et par heure. La masse du volume réactionnel est maintenue constante et le taux de dilution appliqué est de 0,025 h⁻¹. La conduite de l'expérience a été très difficile avec un bouchage répétitif de la pompe de soutirage, le milieu étant très visqueux lorsque le champignon est en croissance. Il y a eu une forte production de champignon et la consigne de pO₂ n'a pas pu être maintenue au dessus de 0%. L'état stationnaire n'a pas pu être atteint. Le k_{L}a du bioréacteur n'était pas suffisant pour amener l'oxygène nécessaire permettant de consommer tout le flux de sucre de l'alimentation. Des k_{L}a de 700 h⁻¹ ont pourtant été mesurés sur eau avec ce réacteur.

### Exemple 7: non-conforme

L'exemple 7 est conduit dans les mêmes conditions que l'exemple 3 à la différence près que la phase en mode "batch" est réalisée avec une concentration en glucose de 65 g/L. La phase a') en mode "fed-batch" et la phase b) de production en mode continu sont réalisées dans les mêmes conditions à la différence près que la solution qui alimente les phases a') et b) est une solution de lactose à 250 g/L acidifiée par ajout d'acide sulfurique H₂SO₄ de sorte que ladite solution ait un pH de 1,5. Les évolutions des concentrations en biomasse cellulaire et en protéines sont présentées sur la figure 4. Sont également représentées sur la figure 4 l'évolution des concentrations en sulfates et en ions ammonium qui sont non limitant. L'expérience ne permet pas d'obtenir une concentration en protéines supérieure à 50 g/L. La concentration en protéines se stabilise à 50 g/L après 400 heures.
Les résultats obtenus montrent l'importance de l'utilisation d'une solution d'hydrolysats hémicellulosiques dans la phase continu de production et que les performances obtenues ne sont pas dues à la levée de la carence en soufre et azote.

Les déterminations analytiques sur le moût final donnent les résultats suivants :
Biomasse : g/L : 23
Protéines : g/L :48,3
UPF : 31,2
β-Glucosidase spécifique : 1,1

### Exemple 8: non-conforme

L'exemple 8 est conduit dans les mêmes conditions que l'exemple 3 à la différence près que la solution d'hydrolysat hémicellulosique issue d'une paille prétraitée par explosion à la vapeur avec imprégnation préalable de H₂SO₄ dans laquelle a été dissout du glucose et du lactose n'est pas stérilisée mais subit une rectification de son pH par ajout de NaOH. Son pH est remonté à 4.
La production de protéines s'arrête après 160 h et reste stable à une concentration proche de 20 g/L.

Les déterminations analytiques sur le moût final donnent les résultats suivants :
Biomasse : g/L : 25,1
Protéines : g/L : 19,8
UPF : 15,8 IU/mL
β-Glucosidase spécifique : 1,2 IU/mg

## Revendications

1. Procédé de production de cellulases et hémicellulases par une souche appartenant à un champignon filamenteux, en bioréacteur agité et aéré comprenant au moins deux phases :
- une phase a) de croissance de ladite souche en présence d'au moins un substrat carboné de croissance en réacteur fermé, ladite phase de croissance étant réalisée avec une concentration en substrat carboné de croissance comprise entre 10 et 90 g/L
- une phase b) continue de production de cellulases dans laquelle au moins un substrat carboné inducteur est alimenté à un débit d'alimentation au moins constant pendant une durée au moins supérieure à 200h, ledit substrat carboné inducteur étant au moins une solution aqueuse d'hydrolysat hémicellulosique issu d'un prétraitement acide d'un substrat lignocellulosique, ladite solution aqueuse d'hydrolysat hémicellulosique ne subissant pas de stérilisation préalable et pas de rectification de pH, ledit pH de la solution aqueuse étant compris entre 0,5 et 3, la masse du volume réactionnel étant maintenu constant par soutirage d'une fraction dudit volume réactionnel, ladite phase b) opérant à un taux de dilution compris entre 0,001 et 0,008 h⁻¹,
dans lequel le substrat carboné inducteur utilisé dans la phase b) est une solution aqueuse d'hydrolysat hémicellulosique issu d'un prétraitement acide d'un substrat lignocellulosique en mélange avec au moins un autre substrat carboné choisi parmi des sucres inducteurs ou non inducteurs,
dans lequel le débit d'alimentation dudit substrat carboné inducteur est compris entre 35 et 140 mg de substrat carboné inducteur par gramme en poids sec de souche et par heure.

2. Procédé selon la revendication 1 dans lequel ladite souche est une souche de *Trichoderma reesei* modifiée par mutation, sélection ou recombinaison génétique.

3. Procédé selon l'une des revendications 1 ou 2 dans lequel le substrat carboné de croissance utilisé dans ladite phase de croissance est choisi le glucose, le lactose, le xylose, les résidus obtenus après fermentation éthanolique des sucres monomères des hydrolysats enzymatiques de substrat lignocellulosique et les extraits de la fraction hémicellulosique sous forme de monomères provenant de substrat lignocellulosique prétraité, utilisé seul ou en mélange.

4. Procédé selon l'une des revendications 1 à 3 dans lequel le prétraitement acide est une hydrolyse acide, une cuisson acide ou une explosion à la vapeur avec imprégnation préalable dudit substrat lignocellulosique avec une solution aqueuse d'acide sulfurique.

5. Procédé selon l'une des revendications 1 à 4 dans lequel la durée de la phase b) continue de production de cellulases est au moins supérieure à 300h.

6. Procédé selon la revendication 5 dans lequel la durée de la phase b) continue de production de cellulases est au moins supérieure à 400h.

7. Procédé selon l'une des revendications 1 à 6 dans lequel le débit d'alimentation est graduellement augmenté dans la phase b).

8. Procédé selon l'une des revendications 1 à 7 dans lequel ladite phase b) opère à un taux de dilution compris entre 0,002 et 0,008h-1.

9. Procédé selon l'une des revendications 1 à 8 dans lequel une phase optionnelle a') de croissance et de production réalisée dans un réacteur à alimentation continue d'au moins un substrat carboné inducteur, au cours de laquelle aucun soutirage du contenu du fermenteur n'est effectué, est mise en oeuvre entre la phase a) et la phase b).

10. Procédé selon la revendication 9 dans lequel ledit substrat carboné inducteur utilisé dans la phase a') est identique au substrat carboné inducteur utilisé dans la phase b) de production.

11. Procédé selon l'une des revendications 9 ou 10 dans lequel la phase a') est mise en oeuvre pendant une durée comprise entre 50 et 150 h.

## Patentansprüche

1. Verfahren zur Herstellung von Cellulasen und Hemicellulasen durch einen Fadenpilzstamm im belüfteten Rühr-Bioreaktor, das mindestens zwei Phasen umfasst:
- eine Phase a) des Wachstums des Stamms in Gegenwart mindestens eines kohlenstoffhaltigen Wachstumssubstrats im geschlossenen Reaktor, wobei die Wachstumsphase mit einer Konzentration des kohlenstoffhaltigen Wachstumssubstrats im Bereich zwischen 10 und 90 g/l durchgeführt wird
- eine Phase b) zur kontinuierlichen Herstellung von Cellulasen, wobei mindestens ein kohlenstoffhaltiges Induktorsubstrat mit einer Zufuhrrate zugeführt wird, die für eine Dauer von mindestens mehr als 200 h konstant ist, wobei das kohlenstoffhaltige Induktorsubstrat mindestens eine wässrige Lösung eines hemicellulosehaltigen Hydrolysats ist, das aus einer Säurevorbehandlung eines lignocellulosehaltigen Substrats erhalten wurde, wobei die wässrige Lösung eines hemicellulosehaltigen Hydrolysats keiner vorherigen Sterilisation und keiner pH-Wert-Korrektur unterzogen wird, wobei der pH-Wert der wässrigen Lösung im Bereich zwischen 0,5 und 3 liegt, wobei die Masse des Reaktionsvolumens durch Entnahme einer Fraktion des Reaktionsvolumen konstant gehalten wird, wobei die Phase b) bei einem Verdünnungsgrad im Bereich zwischen 0,001 und 0,008 h⁻¹ arbeitet,
wobei es sich bei dem in Phase b) verwendeten kohlenstoffhaltigen Induktorsubstrat um eine wässrige Lösung eines hemicellulosehaltigen Hydrolysats handelt, das aus einer Säurevorbehandlung eines lignocellulosehaltigen Substrats, gemischt mit mindestens einem anderen kohlenstoffhaltigen Substrat, ausgewählt aus den Induktor- oder Nichtinduktorzuckern, erhalten wurde, wobei die Zufuhrrate des kohlenstoffhaltigen Induktorsubstrats im Bereich zwischen 35 und 140 mg kohlenstoffhaltiges Induktorsubstrat pro Gramm Trockengewicht des Stamms und pro Stunde liegt.

2. Verfahren nach Anspruch 1, wobei es sich bei dem Stamm um einen durch Mutation, Selektion oder genetische Rekombination modifizierten *Trichoderma-reesei-*Stamm handelt.

3. Verfahren nach einem der Ansprüche 1 oder 2, wobei das in der Wachstumsphase verwendete kohlenstoffhaltige Wachstumssubstrat ausgewählt ist aus Glucose, Lactose, Xylose, Rückständen, die nach der Ethanolfermentation von Monomerzuckern der enzymatischen Hydrolysate von lignocellulosehaltigem Substrat erhalten wurden, und Extrakten der hemicellulosehaltigen Fraktion in Form von Monomeren, die aus vorbehandeltem lignocellulosehaltigem Substrat stammen, die allein oder als Gemisch verwendet werden.

4. Verfahren nach einem der Ansprüche 1 bis 3, wobei es sich bei der Säurevorbehandlung um saure Hydrolyse, saures Kochen oder Dampfexplosion mit vorheriger Imprägnierung des lignocellulosehaltigen Substrats mit einer wässrigen Schwefelsäurelösung handelt.

5. Verfahren nach einem der Ansprüche 1 bis 4, wobei die Dauer der Phase b) zur kontinuierlichen Herstellung von Cellulasen mindestens mehr als 300 h beträgt.

6. Verfahren nach Anspruch 5, wobei die Dauer der Phase b) zur kontinuierlichen Herstellung von Cellulasen mindestens mehr als 400 h beträgt.

7. Verfahren nach einem der Ansprüche 1 bis 6, wobei die Zufuhrrate in der Phase b) nach und nach erhöht wird.

8. Verfahren nach einem der Ansprüche 1 bis 7, wobei die Phase b) mit einem Verdünnungsgrad im Bereich zwischen 0,002 und 0,008 h⁻¹ arbeitet.

9. Verfahren nach einem der Ansprüche 1 bis 8, wobei eine optionale Wachstums- und Herstellungsphase a'), die in einem Reaktor mit kontinuierlicher Zufuhr von mindestens einem kohlenstoffhaltigen Induktorsubstrat durchgeführt wird, in deren Verlauf keine Entnahme des Inhalts des Fermenters durchgeführt wird, zwischen der Phase a) und der Phase b) ausgeführt wird.

10. Verfahren nach Anspruch 9, wobei das in der Phase a') verwendete kohlenstoffhaltige Induktorsubstrat mit dem kohlenstoffhaltigen Induktorsubstrat identisch ist, das in der Herstellungsphase b) verwendet wird.

11. Verfahren nach einem der Ansprüche 9 oder 10, wobei die Phase a') mit einer Dauer im Bereich zwischen 50 und 150 h ausgeführt wird.

## Claims

1. A process for the production of cellulases and hemicellulases using a strain of a filamentous fungus in a stirred and aerated bioreactor, comprising at least two phases:
• a phase a) for growth of said strain in the presence of at least one carbonaceous growth substrate in a closed reactor, said growth phase being carried out with a concentration of carbonaceous growth substrate in the range 10 to 90 g/L;
• a phase b) for the continuous production of cellulases, in which at least one carbonaceous inducer substrate is supplied at a supply rate which is constant over a period of at least more than 200h, said carbonaceous inducer substrate being at least one aqueous hemicellulosic hydrolysate solution obtained from an acid pre-treatment of a lignocellulosic substrate, said aqueous hemicellulosic hydrolysate solution not undergoing prior sterilization and not undergoing pH rectification, said pH of the aqueous solution being in the range 0.5 to 3, the mass of the reaction volume being kept constant by withdrawing a fraction of said reaction volume, said phase b) being operated at a dilution rate in the range 0.001 to 0.008 h-1,
in which the carbonaceous inducer substrate used in phase b) is an aqueous hemicellulosic hydrolysate solution obtained from an acid pre-treatment of a lignocellulosic substrate, as a mixture with at least one other carbonaceous substrate selected from inducer or non-inducer sugars, in which the supply flow rate of said carbonaceous inducer substrate is in the range 35 to 140 mg of carbonaceous inducer substrate per gram of the dry weight of the strain per hour.

2. A process according to claim 1, in which said strain is a strain of *Trichoderma reesei* modified by mutation, selection or genetic recombination.

3. A process according to claim 1 or claim 2, in which the carbonaceous growth substrate used in said growth phase is selected from glucose, lactose, xylose, residues obtained after ethanolic fermentation of monomeric sugars of the enzymatic hydrolysates of the lignocellulosic substrate and extracts of the hemicellulosic fraction in the form of monomers deriving from the pre-treated lignocellulosic substrate, used alone or as a mixture.

4. A process according to one of claims 1 to 3, in which the acid pre-treatment is an acid hydrolysis, acid cooking or steam explosion with prior impregnation of said lignocellulosic substrate with an aqueous sulphuric acid solution.

5. A process according to one of claims 1 to 4, in which the duration of phase b) for the continuous production of cellulases is more than at least 300 h.

6. A process according to claim 5, in which the duration of phase b) for the continuous production of cellulases is more than at least 400 h.

7. A process according to one of claims 1 to 6, in which the supply flow rate is gradually increased in phase b).

8. A process according to one of claims 1 to 7, in which said phase b) being operated at a dilution rate in the range 0.002 to 0.008 h-1.

9. A process according to one of claims 1 to 8, in which an optional phase a') for growth and production in a reactor with a continuous supply of at least one carbonaceous inducer substrate during which no continuous withdrawal from the fermenter occurs, is carried out between phase a) and phase b).

10. A process according to claim 9, in which said carbonaceous inducer substrate used in phase a') is identical to the carbonaceous inducer substrate used in the production phase b).

11. A process according to claim 9 or claim 10, in which phase a') is carried out for a period in the range 50 to 150 h.
